# EUROPEAN PATENT SPECIFICATION

(11) **EP 0 627 915 B1**
(45) Date of publication and mention of the grant of the patent: **13.10.1999**
(21) Application number: 93900844.7
(22) Date of filing: 04.12.1992
(51) Int. Cl.: A61K 31/19, A61K 31/16, A61K 9/00, A61K 31/165

(54) **SPRAYABLE ANALGESIC COMPOSITION AND METHOD OF USE**
SPRÜHFÄHIGE ANALGETISCHE ZUSAMMENSETZUNG UND VERFAHREN ZU IRHER VERWENDUNG
COMPOSITION ANALGESIQUE PULVERISABLE

(43) Date of publication of application: 14.12.1994
(73) Proprietor: MAYOR PHARMACEUTICALS LABORATORIES,INC., Phoenix, AZ 85034 (US)
(72) Inventor: DEIHL, Joseph, A., Paradise Valley, AZ 85253 (US)
(74) Representative: Gilding, Martin John
(86) International application number: US9210452
(87) International publication number: WO9413280

(56) References cited:
- EP-A- 0 069 600
- WO-A-92/00725
- GB-A- 1 209 849
- NL-A- 9 000 237
- US-A- 4 704 406
- US-A- 5 143 731

## Description

This invention relates to sprayable analgesic compositions. More particularly, the invention pertains to compositions for oral administration of analgesic compounds by absorption through the buccal mucosa.

Topical application of analgesic compositions, i.e., by application to and absorption through the skin, is known. For example, the composition for topical application of a salicylate emulsion foot spray is disclosed in a patent to Modderno (U.S. 2,975,097).

Sprayable topical analgesic anti-inflammatory compositions for treating skin rashes, etc. are disclosed in the Saitoh, et al. patent (U.S. 4,775,667).

Aerosol compositions for inhalation therapy, containing analgesics which are absorbed in the bronchioles and alveoli are disclosed in the patent to Porush, et al. (U.S. 2,868,691).

Liquid analgesics for oral administration are also known. For example, see the patent to Haas (U.S. 4,861,797) which discloses palatable liquid ibuprofen compositions.

Sprayable compositions consisting of an arylalkanoic derivative possessing analgesic properties and a solvent, preferably a lower aliphatic alcohol are disclosed in US-A-4,704,406. These compositions are used topically "in closed proximity" of the site of an inflammation. The administration takes place in any case through the skin and not through a mucosa, particularly not through the buccal mucosa.

A sprayable analgesic composition for use on the buccal and vaginal mucosa is disclosed in NL-A-9000237. However this composition is for treating topically ulcerative and erosive diseases of these mucosa. This document does not teach the introduction of the analgesic in the bloodstream thanks to this administration route.

The introduction of an analgesic in the bloodstream by administering an analgesic composition through a mucous membrane in the form of a sublingual tablet or of a nebulizer solution is disclosed in GB-A-1,209,849. A nebulizer solution distinguishes from a sprayable solution in that it must be inhaled.

The sublingual administration of buprenorphine in the form of sublingual administrable liquid compositions is similarly disclosed in EP-A-0 069 600.

It would be highly advantageous to provide compositions in buccal absorption methods for administering analgesics, because such use and methods could be much more convenient for use by the general public, when it is not practical to use tabletted or swallowed liquid compositions. Moreover, compositions and methods for buccal mucosa administration of analgesics would be especially useful to persons who have impaired ability or aversion to swallowing tablets or liquid preparations.

Additionally, it would be advantageous to provide analgesic compositions and methods of administering analgesics which provide a desired physiological effect with the same or lower dosage compared to tabletted or liquid analgesic compositions.

The liquid analgesic compositions of the present invention are conveniently and inexpensively prepared, conveniently administered, and may provide the desired physiological effect at a lower total dose than that obtained by use of prior tabletted or swallowed liquid compositions. The compositions have been found particularly useful by persons who have a limited ability to use oral ingested tablets or liquids or have an aversion to such products. Further, it appears that a desired physiological result, i.e., alleviation of headaches can be obtained by administration of only approximately 1/20th of the dose normally recommended for tabletted analgesics such as acetaminophen.

According to a first aspect, the present invention provides the use of a sprayable analgesic composition in the manufacture of a medicament for use in a method involving introducing an analgesic compound into the bloodstream by absorption through the buccal mucosa by spray application of the composition to the buccal mucosa, the sprayable analgesic composition comprising an analgesic compound and a pharmacologically acceptable liquid carrier for the analgesic compound, whereby the composition is of a viscosity for spray application onto the buccal mucosa.

The sprayable analgesic composition includes an analgesic compound which is capable of introduction into the bloodstream by absorption through the buccal mucosa in a pharmacologically acceptable liquid carrier. The viscosity of the composition is adjusted to permit spray application of the composition to the buccal mucosa. In a preferred embodiment, the analgesic compound is acetaminophen. In another embodiment, the analgesic compound is ibuprofen. In the preferred embodiment, the liquid carrier is an aqueous ethanol liquid.

According to another embodiment of the invention, the above-described composition is contained in a measured dose spray dispenser which delivers a physiologically effective quantity of the composition in one or more, preferably in from 1-5, measured doses.

As used herein, the term analgesic is intended to describe any of the several known analgesics, such as acetaminophen, aspirin, ibuprofen, naproxen and the like, some of which also exhibit anti-inflammatory and/or antipyretic physiological activity.

The compositions preferably also include, in addition to the analgesic and liquid carrier components, other optional ingredients such as surfactants, humectants, preservatives, flavoring agents and other topical pharmaceutical adjuvants and excipients.

The compositions are prepared by art-recognized techniques which are typically used in the preparation of similar sprayable compositions.

### EXAMPLE I

The following example illustrates presently preferred practice of the invention and does not serve as a limitation on the scope of the invention which is limited only by the appended claims.

Having the following compositions:

| Component | Parts by Weight | Description |
|---|---|---|
| SD alcohol | 50 | solvent |
| acetaminophen | 12 | analgesic |
| distilled water | 271 | carrier |
| sorbitol | .5 | surfactant |
| glycerine | 50 | humectant |
| Sorbistat-K | .7 | preservative |
| cyanocobalum | .02 | Vitamin B₁₂ |
| sucrose | 220 | flavor |
| pyridoxine | 3.6 | Vitamin B₆ |
| Tween 80 | 5 | surfactant |
| "Crest" | 3 | flavor |
| EDTA | .5 | preservative |
| fruit juice | 2.5 | flavor |

The above-described composition is packaged in a measured dose spray dispenser containing enough of the composition to provide 240 spray doses containing 1 milligram acetaminophen per spray. (One spray equals 50 microliters.)

### EXAMPLE II

Patients with common headaches are instructed to use the spray dispensers by administering two of the measured dose sprays into the mouth, on the inner cheeks and under the tongue, wait five minutes and then administer two more sprays in the same manner.

Simple headaches are relieved with one or two repetitions of the above procedure.

### EXAMPLE III

Procedures of Examples I and II are repeated, except that the acetaminophen is replaced with ibuprofen. Similar results are obtained.

The sprayable compositions are, desirably, solutions of the active ingredients and other components. However, it is also contemplated that stable suspensions of the active ingredient and other components can be employed.

## Claims

1. Use of a sprayable analgesic composition in the manufacture of a medicament for use in a method involving introducing an analgesic compound into the bloodstream by absorption through the buccal mucosa by spray application of the composition to the buccal mucosa, the sprayable analgesic composition comprising an analgesic compound and a pharmacologically acceptable liquid carrier for the analgesic compound, whereby the composition is of a viscosity for spray application onto the buccal mucosa.

2. The use of a sprayable analgesic composition of Claim 1 wherein said analgesic compound is acetaminophen.

3. The use of a sprayable analgesic composition of Claim 1 wherein said analgesic compound is ibuprofen.

4. The use of a sprayable analgesic composition of Claim 1 wherein said liquid carrier is an aqueous alcoholic liquid.

5. The use of a sprayable analgesic composition of Claim 1 further including a measured dose spray dispenser for delivering a physiologically effective quantity of said composition from about 1-5 measured spray doses.

## Patentansprüche

1. Verwendung einer sprühfähigen analgetischen Zubereitung bei der Herstellung eines Medikaments zur Verwendung im Rahmen einer Methode zur Einführung eines Analgetikums in den Blutstrom durch Absorption über die Mundschleimhaut durch Sprühapplikation der Zubereitung auf die Mundschleimhaut, wobei die sprühfähige analgetische Zubereitung ein Analgetikum und einen pharmakologisch akzeptablen flüssigen Träger für das Analgetikun umfaßt und eine Viskosität zur Sprühapplikation auf die Mundschleimhaut aufweist.

2. Verwendung einer sprühfähigen analgetischen Zubereitung nach Anspruch 1, wobei das Analgetikum aus Acetaminophen besteht.

3. Verwendung einer sprühfähigen analgetischen Zubereitung nach Anspruch 1, wobei das Analgetikum aus Ibuprofen besteht.

4. Verwendung einer sprühfähigen analgetischen Zubereitung nach Anspruch 1, wobei der flüssige Träger aus einer wäßrigalkoholischen Flüssigkeit besteht.

5. Verwendung einer sprühfähigen analgetischen Zubereitung nach Anspruch 1, weiterhin umfassend einen Sprayspender für eine abgemessene Dosis zur Abgabe einer physiologisch wirksamen Menge der Zubereitung aus etwa 1-5 abgemessenen Sprühstößen.

## Revendications

1. utilisation d'une composition analgésique pulvérisable dans la fabrication d'un médicament pour utilisation dans une méthode impliquant l'introduction d'un composé analgésique dans la circulation sanguine par absorption à travers la muqueuse buccale, par pulvérisation de la composition sur la muqueuse buccale, la composition analgésique pulvérisable comprenant un composé analgésique et un liquide vecteur pharmacologiquement acceptable pour la substance analgésique, où la composition est d'une viscosité adaptée à la pulvérisation sur la muqueuse buccale.

2. Utilisation d'une composition analgésique pulvérisable selon la revendication 1 dans laquelle ladite substance analgésique est de l'acétaminophène.

3. Utilisation d'une composition analgésique pulvérisable selon la revendication 1 dans laquelle ladite substance analgésique est de l'ibuprofène.

4. Utilisation d'une composition analgésique pulvérisable selon la revendication 1 dans laquelle ledit liquide vecteur est un liquide alcoolique aqueux.

5. Utilisation d'une composition analgésique pulvérisable selon la revendication 1 comprenant en outre un distributeur-doseur de pulvérisation permettant de délivrer une quantité physiologiquement efficace de ladite composition de environ 1 à 5 doses de pulvérisation mesurées.
